(19) Europäisches Patentamt European Patent Office Office européen des brevets

(11) **EP 4 597 080 A1**

(12) **EUROPEAN PATENT APPLICATION**
published in accordance with Art. 153(4) EPC

(43) Date of publication:
**06.08.2025 Bulletin 2025/32**

(21) Application number: **23873220.0**

(22) Date of filing: **27.09.2023**

(51) International Patent Classification (IPC):
*G01N 21/84* (2006.01)  *G01N 21/25* (2006.01)
*G06T 7/90* (2017.01)  *G06V 10/10* (2022.01)
*B01L 7/00* (2006.01)  *C12Q 1/686* (2018.01)
*G01N 21/17* (2006.01)

(52) Cooperative Patent Classification (CPC):
**B01L 7/00; C12Q 1/686; G01N 21/17; G01N 21/25;
G01N 21/84; G06T 7/90; G06V 10/10**

(86) International application number:
**PCT/KR2023/015053**

(87) International publication number:
**WO 2024/072111 (04.04.2024 Gazette 2024/14)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.09.2022 KR 20220125179**

(71) Applicant: **Optolane Technologies Inc.
Bundang-gu
Seongnam-si, Gyeonggi-do 13494 (KR)**

(72) Inventors:
• **SONG, Hyun-Woo
Seongnam-si Gyeonggi-do 13638 (KR)**

• **KIM, Jae Hun
Seoul 06365 (KR)**
• **EOM, Jaewon
Seongnam-si Gyeonggi-do 13485 (KR)**
• **BYEON, Jaeo
Seongnam-si Gyeonggi-do 13595 (KR)**
• **LEE, Do Young
Seoul 06002 (KR)**
• **CHOI, Kyung-Hak
Yongin-si Gyeonggi-do 17000 (KR)**

(74) Representative: **Schiweck Weinzierl Koch
Patentanwälte Partnerschaft mbB
Ganghoferstraße 68 B
80339 München (DE)**

(54) **DIGITAL REAL-TIME PCR ANALYSIS METHOD**

(57)    The present invention relates to a digital real-time PCR analysis method and aims to provide a digital real-time PCR method that enables digital real-time PCR analysis for samples across a wide concentration range, including both high and low concentration samples that exceed the measurement limits of conventional methods.

[FIG. 22]

EP 4 597 080 A1

**Description**

Technical Field

The present application claims the benefit of priority based on Korean Patent Application No. 10-2022-0125179 filed on September 30, 2022 and Korean Patent Application No. 10-2023-0130511 filed on September 27, 2023, the entire disclosures of which are incorporated as a part of this specification.

The present disclosure relates to a digital real-time PCR analysis method, and more particularly, to a digital real-time PCR analysis method capable of performing digital real-time PCR analysis on high-concentration or low-concentration samples, i.e., on samples with a wide concentration range which exceeds existing measurement limits.

Background Art

Gene amplification technology is an essential process in molecular diagnosis, which is a technology that repeatedly replicates and amplifies a specific base sequence of a small amount of deoxyribonucleic acid (DNA) or ribonucleic acid (RNA) in a sample. Polymerase chain reaction (PCR) is a representative gene amplification technology and consists of three steps of DNA denaturation, primer annealing, and DNA extension, and since each step depends on the temperature of the sample, DNA may be amplified by repeatedly changing the temperature of the sample.

Among PCR analysis, digital PCR is a new PCR technology that detects and quantifies nucleic acids, which creates thousands to millions of partitions ranging from nanoliters to picoliters in size and may calculate the number of target nucleic acids by determining the presence or absence of nucleic acids in each partition. Therefore, it is an absolute quantitative method that may be quantified with a single PCR without having to rely on a standard curve using standard materials for quantification, has high measurement precision, and allows analysis of complex mixtures and linear quantitative detection of results.

The most widely used technology at present is the droplet digital PCR (ddPCR) method that adopts a method of partitioning into droplets, which is a technology that divides a 20 microliter sample into 20,000 droplets and amplifies them. However, the dropletization of the sample must be done using a special device called a droplet generator, and the formed droplets must be transferred to a 96-well PCR plate and then the PCR reaction must be performed. In addition, there is a disadvantage in that the droplets are easy to break, resulting in loss of some of the sample. In addition to the analysis equipment, 5 to 6 other equipment are required, and skilled personnel are also required for analysis, and there is a disadvantage in that it is difficult to measure the real-time curve.

Most of the existing digital PCR technologies, including the above, are end-point methods that measure the fluorescence intensity of each partition after the PCR process is completed. Partitions with high fluorescence intensity are determined as positive, and partitions with low fluorescence intensity are determined as negative. The end-point method may only confirm the presence or absence of target nucleic acids in the partitions, and since it is not possible to know how much is present in each partition, the Poisson probability distribution is applied to calculate the number of target nucleic acids in the sample. This digital PCR technology has two limitations.

First, there are cases where it is difficult to clearly distinguish between positive and negative in each partition. This is the case when the fluorescence intensity of a certain partition is located between the fluorescence intensities of the positive partition and negative partition, making it difficult to determine whether it is positive or negative. If partitions are determined as positive or negative based on criteria arbitrarily set by the user, false positive and false negative errors per partition are included. This may cause problems in the accuracy of quantitative values in low-concentration samples rather than high-concentration samples, i.e., in cases where sensitive detection and quantification are required. In extreme cases, when a reaction appears positive in only one partition, it is very difficult to determine whether it should be classified as positive or negative. This low-concentration accuracy problem may cause the limit of detection (LOD) and limit of quantitation (LOQ) of quantitative diagnostic products to drop. It is difficult to solve this problem even if the number of partitions and the amount of samples are increased to improve the LOD of low-concentration samples. This is because the partitions with intermediate intensities of positive and negative also increase as the number of partitions and samples increases. It is similar to the principle that when the signal intensity increases, the noise intensity also increases.

Second, if all partitions are determined to be positive, the number of target nucleic acids cannot be known because the Poisson probability distribution cannot be used. In this case, the sample is usually serially diluted and retested, and the number of target nucleic acids is calculated using the Poisson probability distribution in the test that produces a negative partition. In other words, the end-point method has a limited dynamic range that may be measured in a single test, which is determined by the number of partitions and the volume of the partitions. For example, if there are 1 nanoliter per partition and 20,000 partitions, the dynamic range for detecting targets is from 1 to a minimum of 100,000 and a maximum of 300,000. If there are 1 million high-concentration targets in 20 microliters of a sample, it cannot be quantified in a single test.

Disclosure of the Invention

Technical Goals

The present disclosure relates to a digital real-time PCR analysis method, and is to provide a digital real-time PCR analysis method capable of performing digital real-time PCR analysis on high-concentration or low-concentration samples, i.e., on samples with a wide concentration range that exceeds existing measurement limits.

Technical objects to be achieved by the present disclosure are not limited to the technical objects mentioned above, and other technical objects not mentioned may be clearly understood by those skilled in the art to which the present disclosure pertains from the description below.

Technical Solutions

A digital real-time PCR analysis method of the present disclosure uses a cartridge for digital real-time PCR including a microfluidic chamber in which an analysis target sample of a liquid phase is stored; a well array attached to a bottom surface of the microfluidic chamber and supplied with the analysis target sample from the microfluidic chamber; and a CMOS photosensor array located on a bottom surface of the well array and configured to capture a reaction image of the analysis target sample filled in a plurality of partitions provided in the well array in real-time, wherein the method may include,

a sample injection step (S10) of injecting the analysis target sample of the microfluidic chamber into each of the plurality of partitions;

a raw data acquisition step (S20) of acquiring raw data by capturing the reaction image of the analysis target sample filled in the plurality partitions in real-time through the CMOS photosensor array;

a cycle threshold (Ct) extraction step (S30) of extracting a plurality of Ct values for the plurality of partitions, respectively, from the raw data;

an individual partition target number obtaining step (S40) of obtaining a plurality of target numbers for the plurality of partitions, respectively, based on the plurality of Ct values; and

a final output step (S50) of obtaining a target concentration in the analysis target sample based on the plurality of target numbers.

Advantageous Effects

The digital real-time PCR analysis method of the present disclosure may provide a digital real-time PCR analysis method capable of performing digital real-time PCR analysis on high-concentration or low-concentration samples, i.e., on samples with a wide concentration range that exceeds existing measurement limits.

The digital real-time PCR analysis method of the present disclosure may implement digital PCR that provides real-time graphs for individual partitions, thereby eliminating false positive or false negative errors.

The digital real-time PCR analysis method of the present disclosure may achieve the same effect as repeatedly evaluating digital real-time PCR tens of thousands of times with one cartridge, and may obtain the initial concentration in the sample by calculating the number of targets present in each unit compartment based on the values measured in each unit compartment.

Brief Description of Drawings

FIG. 1 is a perspective view for schematically explaining a cartridge for digital real-time PCR according to an embodiment of the present disclosure.

FIG. 2 is an exploded perspective view of the cartridge for digital real-time PCR illustrated in FIG. 1.

FIG. 3 is a perspective view for schematically explaining an example of a well array shape illustrated in FIG. 2.

FIGS. 4A and 4B are diagrams showing digital real-time PCR results.

FIG. 5A is a front perspective view for schematically explaining a microfluidic chamber illustrated in FIG. 2, FIG. 5B is a back perspective view for schematically explaining the microfluidic chamber illustrated in FIG. 2, and FIG. 5C is an exploded perspective view of the microfluidic chamber illustrated in FIG. 2.

FIG. 6 is a cross-sectional view for schematically explaining a PCR module illustrated in FIG. 2.

FIGS. 7 to 21 are schematic cross-sectional views illustrating use of a cartridge for digital real-time PCR.

FIG. 22 is a block diagram illustrating a digital real-time PCR analysis method of the present disclosure.

Best Mode for Carrying Out the Invention

A digital real-time PCR analysis method of the present disclosure may use a cartridge for digital real-time PCR including a microfluidic chamber in which an analysis target sample of a liquid phase is stored; a well array attached to a bottom surface of the microfluidic chamber and supplied with the analysis target sample from the microfluidic chamber; and a CMOS photosensor array located on a bottom surface of the well array and configured to capture a reaction image of the analysis target sample filled in a plurality of partitions provided in the well array in real-time.

A digital real-time PCR analysis method may include:

a sample injection step (S10) of injecting the analysis target sample of the microfluidic chamber into each of the plurality of partitions;

a raw data acquisition step (S20) of acquiring raw data by capturing the reaction image of the analysis target sample filled in the plurality partitions in real-time through the CMOS photosensor array;

a cycle threshold (Ct) extraction step (S30) of extracting a plurality of Ct values for the plurality of partitions, respectively, from the raw data;

an individual partition target number obtaining step (S40) of obtaining a plurality of target numbers for the plurality of partitions, respectively, based on the plurality of Ct values; and

a final output step (S50) of obtaining a target concentration in the analysis target sample based on the plurality of target numbers.

In the sample injection step (S10) of the digital real-time PCR analysis method of the present disclosure, the microfluidic chamber may be formed of polydimethylsiloxane (PDMS) material.

In the sample injection step (S10) of the digital real-time PCR analysis method of the present disclosure, when the analysis target sample is injected into each of the plurality of partitions, the well array may be completely in contact with the CMOS photosensor array.

The raw data acquisition step (S20) of the digital real-time PCR analysis method of the present disclosure may be performed after confirming that all of the plurality of partitions are filled with the analysis target sample, and in the raw data acquisition step (S20), images for the plurality of partitions may be acquired as the raw data.

In the raw data acquisition step (S20) of the digital real-time PCR analysis method of the present disclosure, the raw data may be collected at least once per cycle.

In the Ct extraction step (S30) of the digital real-time PCR analysis method of the present disclosure, a fluorescence intensity function with fluorescence intensity as a dependent variable and time or cycle number as an independent variable may be acquired for each of the plurality of partitions from the raw data, and the Ct value may be a time or cycle number value at which a second differentiation value of the fluorescence intensity function is at a maximum.

In the target number obtaining step (S40) of the digital real-time PCR analysis method of the present disclosure, each of the plurality of target numbers for the plurality of partitions may be obtained by the following Equation 1.

[Equation 1]

$$N_i = 10^{\frac{Ct_i - Ct_{ref}}{a}},$$

where $N_i$ is the target number in an $i^{th}$ partition,

$Ct_i$ is the Ct value of the $i^{th}$ partition,

$Ct_{ref}$ is determined by at least one or more of an amplicon number required to measure Ct and a fluorescence efficiency of a probe, and

a is a constant determined by a PCR efficiency.

In the target number obtaining step (S40) of the digital real-time PCR analysis method of the present disclosure, the $Ct_{ref}$ may be obtained by the following Equation 2.

[Equation 2]

$$Ct_{\mathrm{ref}} = a \times \log\left(\frac{1}{V_{unit}}\right) + Ct_0$$

,

where $Ct_0$ may be a Ct value acquired from a standard sample.

A concentration of the standard sample of the digital real-time PCR analysis method of the present disclosure may be $10^7$ copies/rxn to $10^8$ copies/rxn.

In the final output step (S50) of the digital real-time PCR analysis method of the present disclosure, a concentration Con of the analysis target sample may be obtained by the following Equation 3.

[Equation 3]

$$Con = \sum_{i=1}^{n} N_i$$

,

where n is a total number of the plurality of partitions.

In the final output step (S50) of the digital real-time PCR analysis method of the present disclosure, negativity or positivity of the analysis target sample may be determined based on the Con value.

Modes for Carrying Out the Invention

Hereinafter, embodiments according to the present disclosure will be described in detail with reference to the accompanying drawings. In this process, the size or shape of components shown in the drawings may be exaggerated for clarity and convenience of explanation. In addition, terms specifically defined in consideration of the configuration and operation of the present disclosure may vary according to the intentions or customs of users and operators. Definitions of these terms should be made based on the content throughout this specification.

In the description of the present disclosure, it should be noted that the orientation or positional relationship indicated by the terms such as "center", "upper", "lower", "left", "right", "vertical", "horizontal", "inner side", "outer side", "one surface", and "other surface" is based on the orientation or positional relationship shown in the drawing or the orientation or positional relationship normally arranged when using a product of the present disclosure, and it is not to be construed as limiting the present disclosures because it is intended merely for explanation and brief description of the present disclosure and does not suggest or imply that the device or element shown must necessarily be configured or operated in a specific orientation with a specific orientation.

Terms such as first and second may be used to describe various components, but the components should not be limited by the terms. The terms are used only for the purpose of distinguishing one component from another. For example, a first component may be referred to as a second component without departing from the scope of the present disclosure, and similarly, the second component may also be referred to as the first component. Singular expressions include plural expressions unless the context clearly means otherwise.

In this specification, it should be understood that terms such as "comprise", "include", or "have" are intended to designate the presence of features, numbers, steps, operations, components, parts, or combinations thereof described in the specification, but do not preclude the possibility of the presence or addition of one or more other features, numbers, steps, operations, components, parts, or combinations thereof.

In addition, unless otherwise defined, all terms used herein, including technical or scientific terms, have the same meaning as those generally understood by a person skilled in the art to which the present disclosure pertains. Terms defined in dictionaries generally used should be construed to have meanings matching contextual meanings in the related art and are not to be construed as an ideal or excessively formal meaning unless otherwise defined herein.

Hereinafter, the present disclosure will be described in more detail with reference to the attached drawings. For identical components in the drawings, the same reference numerals are used, and redundant descriptions of identical components are omitted.

The present disclosure relates to a structure for mounting samples, sample volumes, or reaction volumes in a predetermined arrangement on a substrate, and more particularly, to mounting samples in a predetermined arrangement of respective reaction sited within a substrate.

In various embodiments, devices, apparatuses, systems, and methods are provided for mounting samples within an article used to detect targets in a large number of small-volume samples. Such targets may be any suitable biological target, including DNA sequences (including cell-free DNA), RNA sequences, genes, oligonucleotides, molecules, proteins, biomarkers, cells (e.g., circulating tumor cells), or other suitable target biomolecules, but are not limited thereto. In various embodiments, such biological components may be used in conjunction with various PCR, qPCR, and/or dPCR methods and systems for applications such as prenatal diagnosis, multiplex dPCR, virus detection, and quantitative normalization, genotyping, sequence verification, mutation detection, genetic biology, rare allele detection, and copy number variation detection, etc.

While generally applicable to quantitative polymerase chain reaction (qPCR) where a large number of samples are processed, it should be appreciated that any suitable PCR method may be used in accordance with various embodiments described herein. Suitable PCR methods include, for example, digital PCR, allele-specific PCR, asymmetric PCR, ligationmediated PCR, multiplex PCR, nested PCR, qPCR, casting PCR, genome walking, bridge PCR, but are not limited thereto.

As described below according to various embodiments described herein, reaction sites include, for example, through-holes, sample retention regions, wells, indentations, spots, cavities, and reaction chambers, but are not limited thereto.

The various embodiments described herein are particularly suitable for digital PCR (dPCR). In digital PCR, each sample of a solution embedded with a relatively small number of target polynucleotide or nucleic acid sequences may be further divided into a large number of smaller samples, whereby each sample typically contains one molecule of the target nucleotide sequence or may not contain the target nucleotide sequence. Subsequently, when samples are thermally cycled in a PCR protocol, procedure, or experiment, samples containing the target nucleotide sequence are amplified and generate a positive detection signal, whereas samples not containing any target nucleotide sequence are not amplified and generate no detection signal. Using Poisson statistics, the number of target nucleotide sequences in the original solution may be correlated to the number of samples generating a positive detection signal.

For a typical dPCR protocol, procedure, or experiment, there is an advantage in that the initial sample solution may be divided into tens or hundreds of thousands of samples, i.e., samples having a volume of a few nanoliters, one or approximately one nanoliter, or less than one nanoliter, respectively, in a simple and cost-effective manner. Since the number of target nucleotide sequences may be very small, it may also be important in such environments where the entire contents of the initial solution are embedded in consideration of multiple reaction sites.

Embodiments described herein address these and other dPCR design constraints by distributing the initial sample solution to multiple reaction sites in a manner that considers all, or all of the essentials, of the sample solution.

Because of high throughput PCR analysis and dPCR methods, a strategy using an array format to reduce the reaction volume of the liquid sample while increasing the number of reactions performed at one time may also be applied. The array of reaction volumes of the liquid sample may be a substrate having multiple reaction sites. The reaction sites may be through holes, wells, recesses, points, cavities, reaction chambers, or any structure capable of holding a sample according to the various embodiments described herein, but are not limited thereto. In some embodiments, the through holes or wells may be tapered in diameter.

Reducing the reaction volume of the liquid sample so that more reactions may be performed within a given region may also increase the density of the reaction volumes. For example, a predetermined array of reaction sites configured with a diameter of 300 $\mu$m through through-holes in the substrate may include a reaction volume of about 30 nL. For example, by reducing the size of each through hole in the array to a diameter of 60 to 70 $\mu$m, each reaction volume may be, for example, 100 pL of liquid sample. Reaction volumes according to various embodiments described herein may be from about 1 pL to 30 nL of liquid sample. Furthermore, the dynamic range may be increased by using a dilution of the liquid sample of greater than or equal to 1.

A digital real-time PCR analysis method of the present disclosure may use a cartridge for digital real-time PCR which includes:

a microfluidic chamber in which an analysis target sample of a liquid phase is stored;
a well array attached to a bottom surface of the microfluidic chamber and supplied with the analysis target sample from the microfluidic chamber; and
a CMOS photosensor array located on a bottom surface of the well array and configured to capture a reaction image of the analysis target sample filled in a plurality of partitions provided in the well array in real-time.

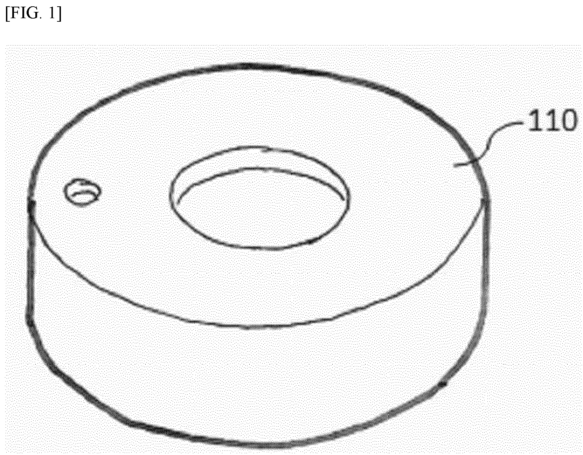

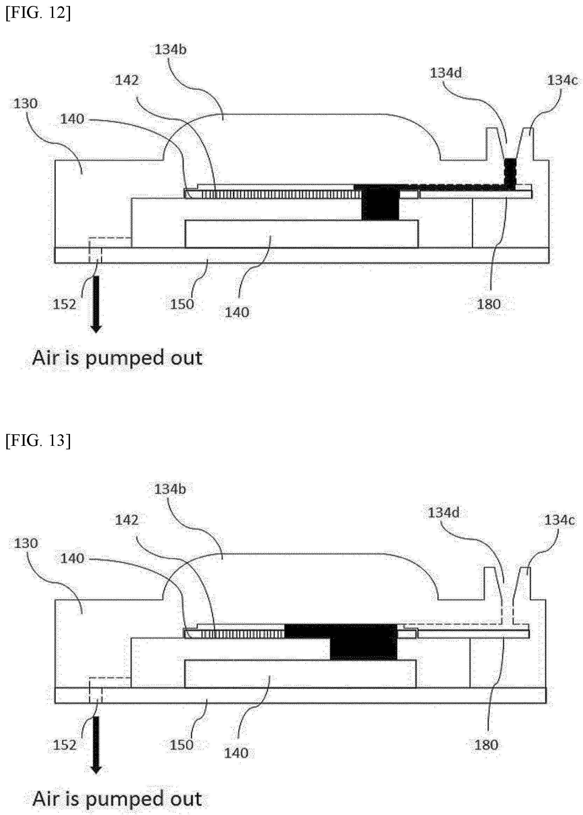

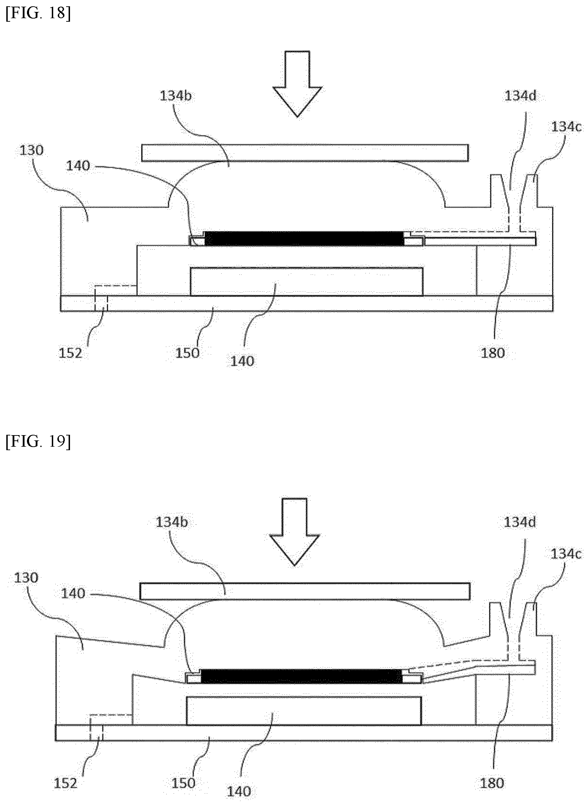

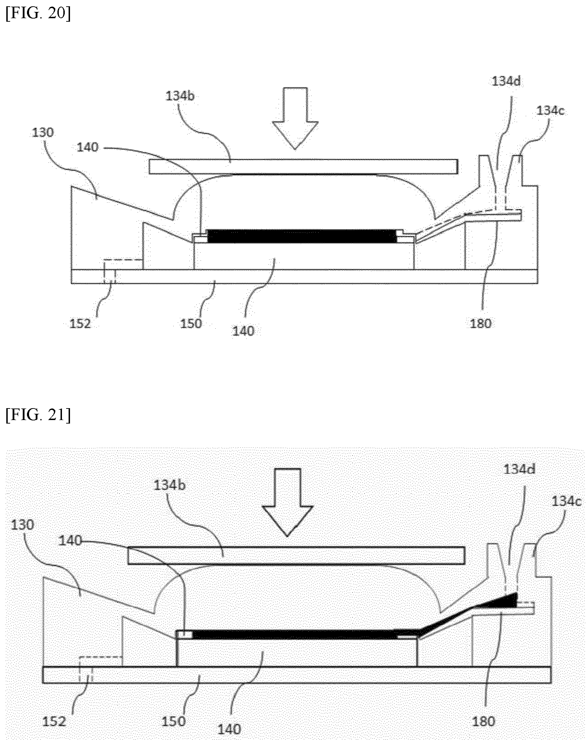

Hereinafter, with reference to FIGS. 1 to 23, a cartridge for digital real-time PCR used in the digital real-time PCR analysis method of the present disclosure will be described.

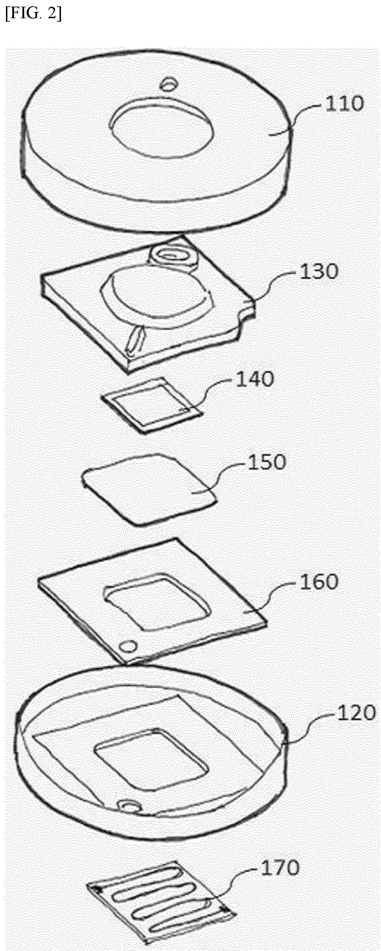

FIG. 1 is a perspective view for schematically explaining a cartridge for digital real-time PCR according to an embodiment of the present disclosure. FIG. 2 is an exploded perspective view of the cartridge for digital real-time PCR illustrated in FIG. 1.

Referring to FIGS. 1 and 2, a cartridge for digital real-time PCR according to an embodiment is a cartridge-type inspection module which includes an upper case 110, a bottom case 120, a microfluidic chamber 130, a well array 140, a CMOS photosensor array 150, and a PCB 160. In the present embodiment, the cartridge for digital real-time PCR may be detachably coupled to the reader system of the digital real-time PCR equipment. In the present embodiment, the microfluidic chamber 130, the well array 140, the CMOS image sensor 150, and the PCB 160 may define a PCR module.

The upper case 110 includes a donut-shaped cover body including an upper hole formed in a central region and a window member disposed in the upper hole. The upper case 110 is fastened to the bottom case 120. In the present embodiment, the upper case 110 and the bottom case 120 are illustrated as having a flat cylindrical shape, but various shapes are possible. The window member may include a transparent material. The window member may include a PDMS material.

The bottom case 120 accommodates the microfluidic chamber 130, the well array 140, the CMOS photosensor array 150, and the PCB 160, and is fastened to the upper case 110. The bottom case 120 and the upper case 110 may be fastened in a hook manner.

The microfluidic chamber 130 includes a membrane switch protruding upward and an inlet formed on one side of the membrane switch for injecting a liquid sample. A lower edge region of the microfluidic chamber 130 is in contact with an edge region of the PCB 160. A space having a recessed shape is formed in a lower central region of the microfluidic chamber 130 to accommodate the CMOS photosensor array 150 and the well array 140 mounted on the PCB 160. The microfluidic chamber 130 may be formed of a material such as PDMS. The microfluidic chamber 130 has flexibility, transparency, PCR compatibility, and low autofluorescence, and is injection moldable.

The well array 140 is attached to a lower surface of the microfluidic chamber 130 and is disposed on the CMOS photosensor array 150. The well array 140 may be formed of an etched silicon material. The well array 140 includes a plurality of microwells (approximately 1,000 to 100,000) that serve as PCR reaction sites.

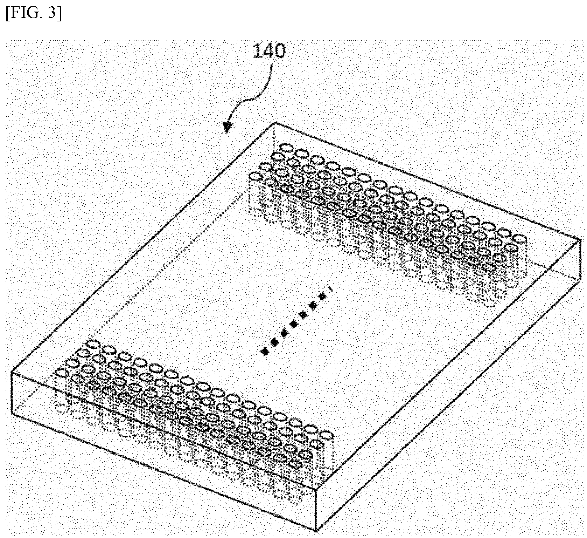

FIG. 3 is a perspective view for schematically explaining an example of a well array shape illustrated in FIG. 2. Although the shape of the microwell in FIG. 3 is illustrated as being circular, various shapes such as hexagons, squares, etc., are possible.

Referring to FIG. 3, the shape, size, and volume of the microwell may be adjusted. The microwell has a shape in which the upper and lower parts are penetrated. In the present embodiment, the microwell may have a thickness of less than 700 $\mu$m and a pitch of less than 150 $\mu$m. The microwell may have various shapes, such as hexagonal shape, circular shape, etc. The well array 140 may be treated with a hydrophilic coating. The well array 140 may be attached to the microfluidic chamber 130 by plasma or heat or UV curing adhesive.

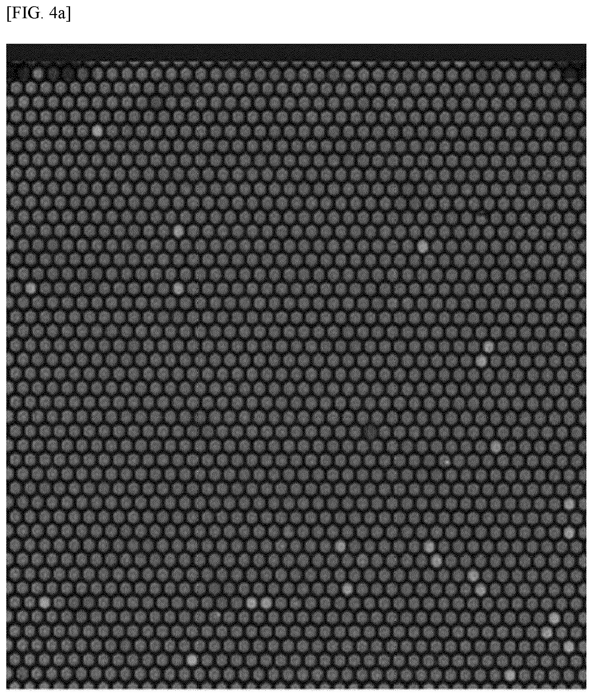

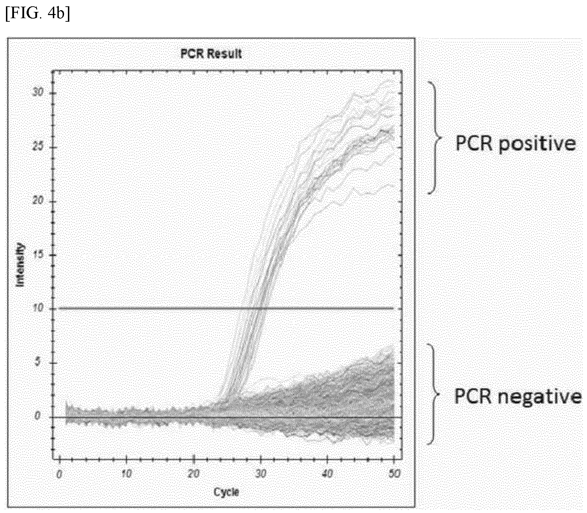

Referring again to FIGS. 1 and 2, the CMOS photosensor array 150 is disposed below the well array 140 and captures PCR reaction products in real-time from the plurality of microwells of the well array 140. Specifically, the CMOS photosensor array 150 is disposed to correspond to a substrate hole formed in the PCB 160 and receives light emitted from the well array 140 to measure reaction products of PCR performed in a PCR device, as illustrated in FIG. 4A. The measured PCR reaction products may be analyzed in a form of a graph, etc., as illustrated in FIG. 4B.

FIGS. 4A and 4B are diagrams showing digital real-time PCR results. In particular, FIG. 4A is a photograph showing PCR positive/negative results in a well array, and FIG. 4B is a graph for measuring fluorescence in real-time in each microwell to determine whether the corresponding well is positive or negative.

Referring again to FIG. 1 and FIG. 2, a thin layer of a material such as PDMS may be coated on the upper surface of the CMOS photosensor array 150 to form a bottom of the microwell after sealing.

The PCB 160 accommodates the CMOS image sensor 150. A vent hole 152 for vacuum processing may be formed in the PCB 160 so that a liquid sample introduced through the inlet is quickly provided to the well array 140. The vent hole 152 gets through to the space between the CMOS photosensor array 150 and the well array 140.

In the present embodiment, the cartridge for digital real-time PCR may further include a heater 170 for thermal cycling. As used herein, thermal cycling may include steps using a thermal cycler, isothermal amplification, thermal convention, infrared mediated thermal cycling, or helicase dependent amplification. In some embodiments, the chip may be integrated with a built-in heating element. In various embodiments, the chip may be integrated with semiconductors. Detection of a target according to various embodiments may include, for example, fluorescence detection, positive or negative ion detection, acidity (pH) detection, voltage detection, or current detection, alone or in combination, but is not limited thereto.

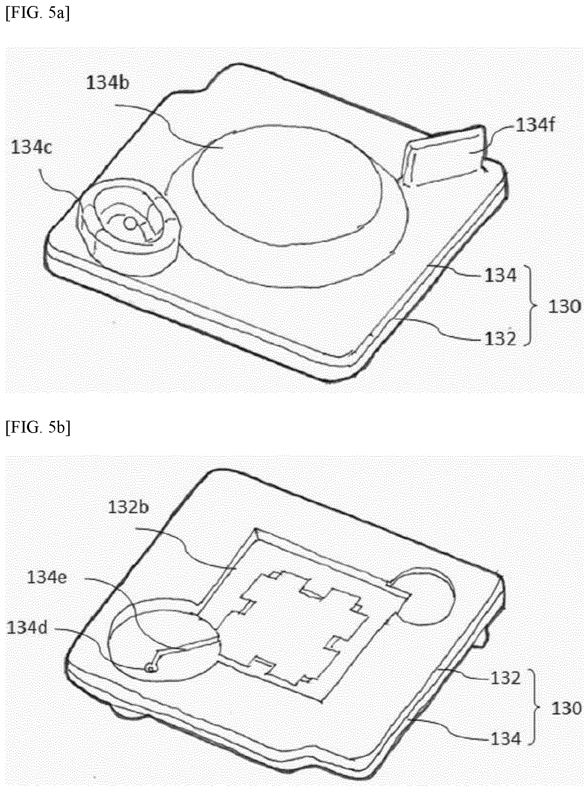

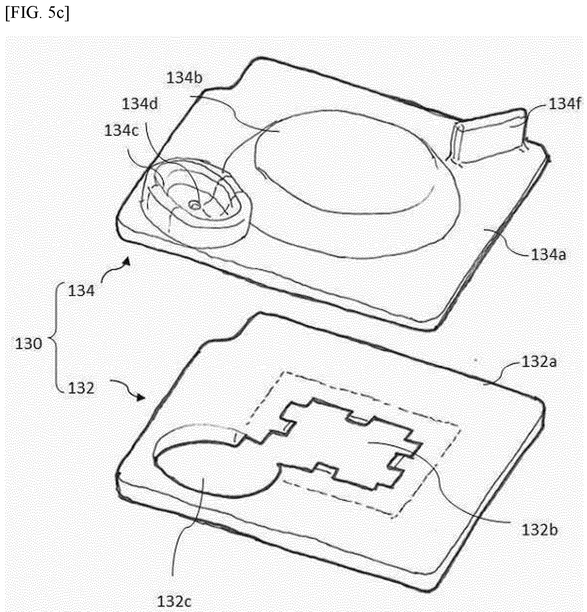

FIG. 5A is a front perspective view for schematically explaining the microfluidic chamber 130 illustrated in FIG. 2, FIG. 5B is a back perspective view for schematically explaining the microfluidic chamber 130 illustrated in FIG. 2, and FIG. 5C is an exploded perspective view of the microfluidic chamber 130 illustrated in FIG. 2.

Referring to FIG. 2 to FIG. 5C, the microfluidic chamber 130 according to the present embodiment includes a square-shaped base member 132 and a square-shaped top member 134 disposed on the base member 132. In the present embodiment, the base member 132 and the top member 134 are illustrated as being physically separated, but may be configured as an integral form.

The base member 132 includes a square-shaped first flat member 132a, a square-shaped support hole 132b formed in a central region of the first flat member 132a, and a circular-shaped flat hole 132c formed in a corner region of the first flat member 132a. In the support hole 132b, guide protrusions protruding toward the central region may be formed to define a square sawtooth shape.

The top member 134 includes a square-shaped second flat part 134a, a plate-shaped membrane switch 134b, and a closed loop-shaped inlet part 134c. The second flat part 134a is in close contact with the upper surface of the first flat part 132a. The membrane switch 134b is disposed in the central region of the second flat part 134a and protrudes upward. The lower region of the membrane switch 134b corresponds to the support hole 132b of the base member 132. Accordingly, a space having a recessed shape is formed at the bottom part of the microfluidic chamber 130. The CMOS image sensor 150 and the well array 140 may be accommodated in the recessed space.

The inlet part 134c has a fence shape and protrudes upward on one side of the membrane switch 134b to block the liquid sample from flowing out to the outside. An inlet 134d is formed in the central region of the inlet part 134c in the vertical direction of the base member 132.

A micro flow path 134e is formed in a region connecting the inlet 134d of the inlet part 134c and a bottom edge region of the membrane switch 134b. The liquid sample introduced into the inlet 134d of the inlet part 134c reaches the bottom edge region of the membrane switch 134b through the micro flow path 134e.

The top member 134 may further include a grip part 134f that protrudes upward from the observer's perspective on the other side of the membrane switch 134b. The grip part 134f is disposed to face the inlet part 134c with respect to the membrane switch 134b. The height of the grip part 134f may be higher than the height of the inlet part 134c. The height of the inlet part 134c and the height of the membrane switch 134b are the same.

As described above, the inlet 134d and the micro flow path 134e get through to each other in the microfluidic chamber 130. When a liquid sample is introduced through the inlet 134d, the liquid sample falls into the space formed below the membrane switch 134b through the micro flow path 134e. In addition, when the membrane switch 134b is pressed, the liquid sample may be completely filled into each of the plurality of microwells of the well array 140 exposed through the micro flow path 134e.

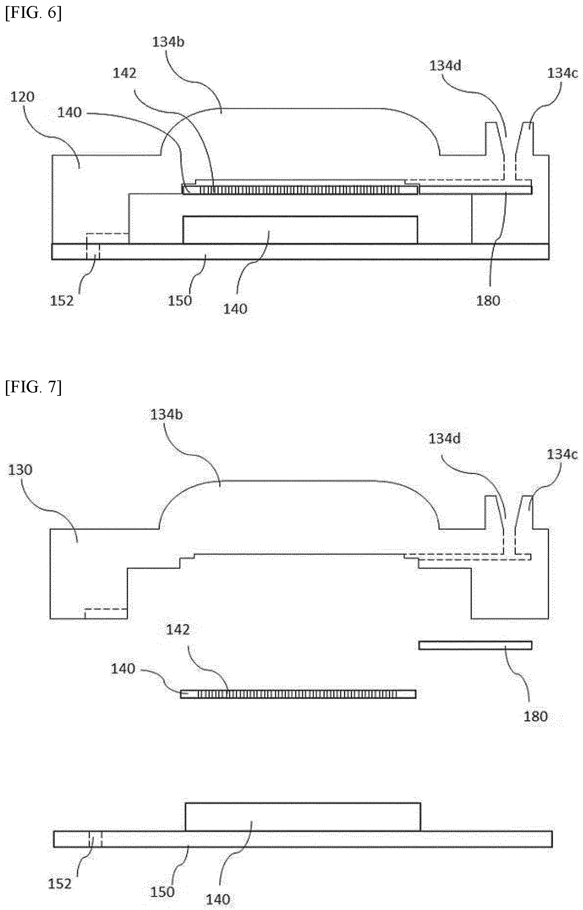

FIG. 6 is a cross-sectional view for schematically explaining a PCR module illustrated in FIG. 2. FIG. 7 is an exploded cross-sectional view for schematically explaining the PCR module illustrated in FIG. 6.

Referring to FIGS. 2 to 7, the PCR module according to an embodiment of the present disclosure includes the microfluidic chamber 130, the well array 140, the CMOS photosensor array 150, and the PCB 160.

A space having a recessed shape is formed in a bottom region of the microfluidic chamber 130 to accommodate the CMOS photosensor array 150 and the well array 140 disposed on the PCB 160. Since the microfluidic chamber 130 has been described with reference to FIGS. 5A to 5C, its description will be omitted.

The well array 140 is attached to the lower surface of the microfluidic chamber 130. The well array 140 is disposed on the CMOS photosensor array 150 and inserted into the substrate hole formed in the PCB 160. The well array 140 includes a plurality of microwells 142. The shape, size, number, etc., of the microwells 142 may vary. An analysis sample such as a powder sample or a liquid sample may be accommodated in each of the plurality of microwells 142. The analysis sample is a specific component for analyzing a biological material. In other words, the analysis sample refers to a component for quantitative or qualitative analysis of a specific biological material, such as protein, DNA, RNA, etc., such as a primer, probe, antibody, aptamer, DNA or RNA polymerase, etc., and in particular, refers to a component necessary for performing real-time polymerase chain reaction, constant-temperature enzymatic reaction, or ligase chain reaction (LCR), etc.

The CMOS photosensor array 150 is disposed below the well array 140 and captures images of PCR reaction products performed in the plurality of microwells 142 of the well array 140 in real-time. The CMOS photosensor array 150 is disposed by being inserted into the substrate hole formed in the PCB 160. The CMOS photosensor array 150 receives the emitted light and captures images of PCR reaction products performed in a PCR device in real-time. In other words, the CMOS photosensor array 150 detects fluorescence (emission light) generated from a plurality of probes by the excitation light. The detection of the fluorescence described above may be performed by a time separation method or may be performed by a wavelength separation method.

In the case of the time separation method described above, as a fluorescent substance emits emission light in response to the excitation light, the fluorescence sensor array or a single sensor constituting the array detects the emission light passing through an emission filter and obtains a time constant of the detected emission light to detect fluorescence.

In the case of the wavelength separation method described above, as a fluorescent material emits emission light in response to the excitation light, the fluorescent sensor array or a single sensor constituting the array detects the emission light passing through an emission filter and detects fluorescence through spectrum analysis of the detected emission light.

The PCB 160 is disposed below the microfluidic chamber 130 and houses the mounted CMOS photosensor array 150. The PCB 160 is disposed so as to be in contact with the bottom edge region of the microfluidic chamber 130. A vent hole 152 is formed in a portion of the PCB 160 that is in contact with the bottom edge region of the microfluidic chamber 130.

The vent hole 152 gets through to the space between the CMOS photosensor array 150 and the well array 140.

When a liquid sample is introduced into the inlet 134d, air is pumped through a vacuum device (not shown) connected to the vent hole 152. As the air is pumped, the liquid sample injected into the inlet 134d is provided to a portion of the well array 140, an upper portion of the portion, and a lower portion of the portion, via the micro flow path 134e formed in the microfluidic chamber 130.

In the present embodiment, a sticker 180 forming the bottom of a micro flow path 134e formed in the microfluidic chamber 130 may be further included. By attaching the sticker 180, the liquid sample introduced into the micro flow path 134e may be provided to the well array 140 intact without flowing out to other regions. The thickness of the sticker 180 may be the same as the thickness of the well array 140.

In the present embodiment, the PCR module may further include a light providing part (not shown) disposed to irradiate excitation light toward probes accommodated in each of the plurality of microwells 142 of the well array 140. In an embodiment, the light providing part may include a light source that emits light, such as a light emitting diode (LED) light source, a laser light source, etc. The light emitted from the light source passes through or is reflected by the microwell 142 of the well array 140, and in this case, the CMOS photosensor array 150 may detect the light signal generated by nucleic acid amplification.

In the present embodiment, the PCR module may further include an emission filter (not shown) that performs the function of selecting light having a predetermined wavelength. The emission filter may be disposed on the CMOS photosensor array 150.

Hereinafter, an explanation is provided using drawings that sequentially illustrate a method of using a cartridge for digital real-time PCR according to an embodiment of the present disclosure. For the convenience of explanation, illustrations of the upper case 110 (shown in FIG. 2) and the bottom case 120 (shown in FIG. 2) are omitted.

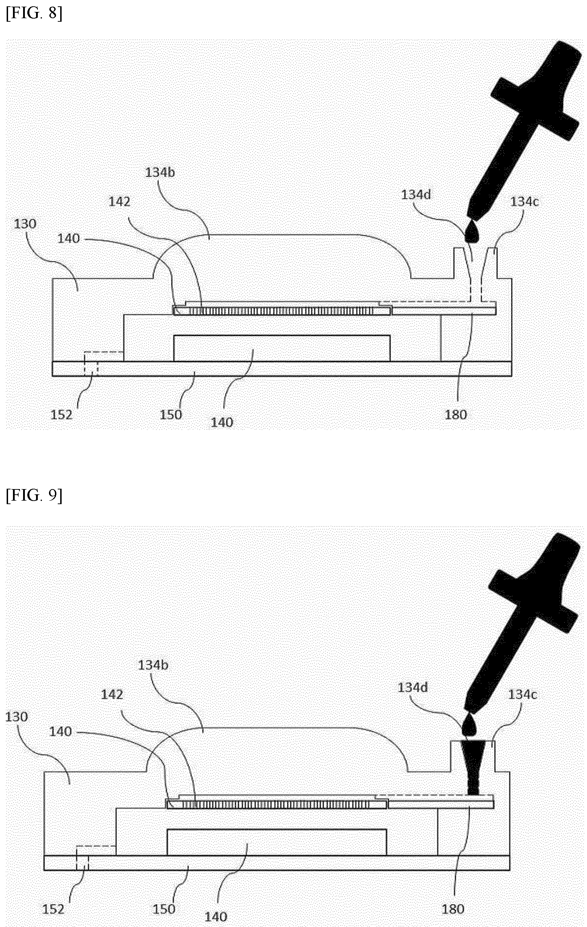

FIGS. 8 to 16 are cross-sectional views illustrating a process in which a liquid sample is injected into the well array 140.

Referring to FIG. 8, an empty PCR module is disposed on a flat location.

Referring to FIG. 9, a liquid sample is pipetted into the inlet 134d of the microfluidic chamber 130. It is preferable that the amount of the liquid sample pipetted into the inlet 134d is an amount that may fill the space between the well array 140 and the microfluidic chamber 130 and the plurality of microwells 142 of the well array 140. The liquid sample pipetted into the inlet 134d cannot flow into the micro flow path 134e (shown in FIG. 5B) due to the resistance of the air existing in the micro flow path 134e.

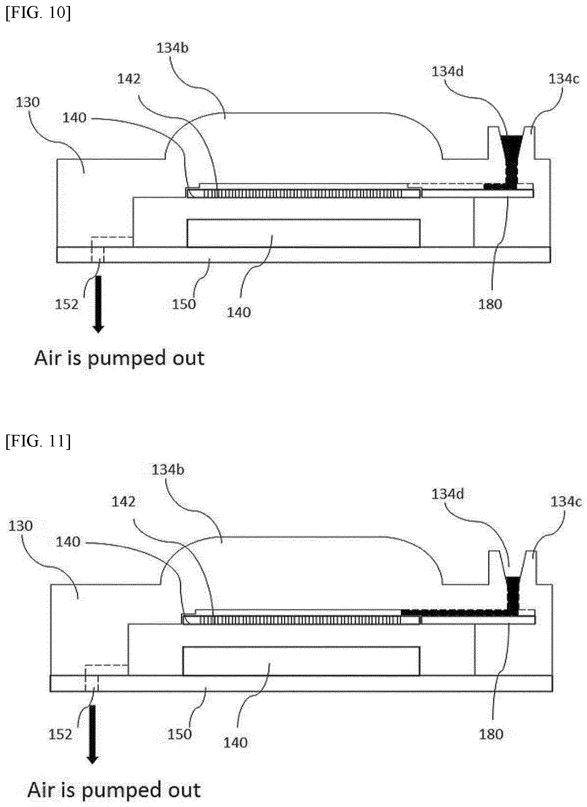

Referring to FIGS. 10, 11, 12, and 13, after the liquid sample is pipetted into the inlet 134d, a negative pressure is formed by a vacuum device (not shown) connected to the vent hole 152.

Accordingly, air is pumped through the vent hole 152, and the liquid sample pipetted into the inlet 134d is provided to the well array 140 along the micro flow path 134e. The liquid sample provided to the well array 140 reaches up to the CMOS photosensor array 150 through the microwell 142 near the end part of the micro flow path 134e. Through the pumping of such vacuum device, the liquid sample may be provided to the plurality of microwells more quickly. In addition, through the pumping of the vacuum device, air that may exist in the corner or edge region of the well array may be removed more easily.

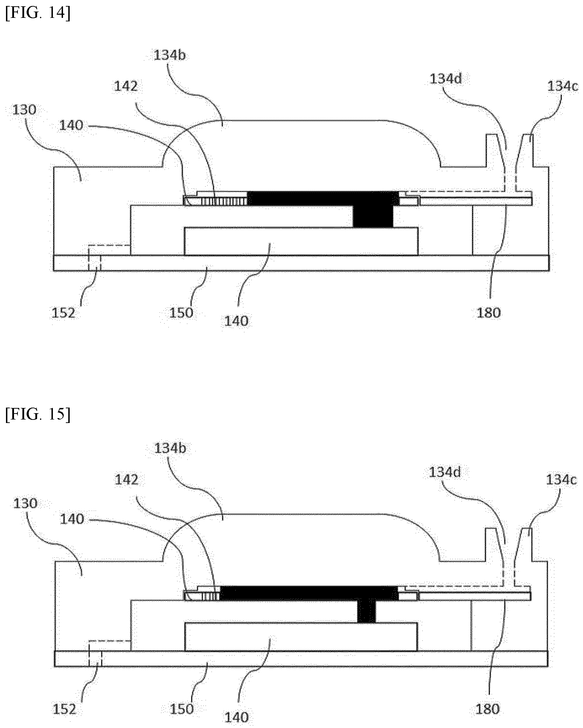

Referring to FIGS. 14, 15, and 16, the vacuum device connected to the vent hole 152 is stopped from operating. As the negative pressure by the vacuum device is relieved, the liquid sample that has reached the lower space of the well array 140 is gradually drawn up to the upper space of the well array 140 by the capillary force.

Accordingly, the liquid sample is filled in the space between the well array 140 and the microfluidic chamber 130 and the plurality of microwells 142 of the well array 140.

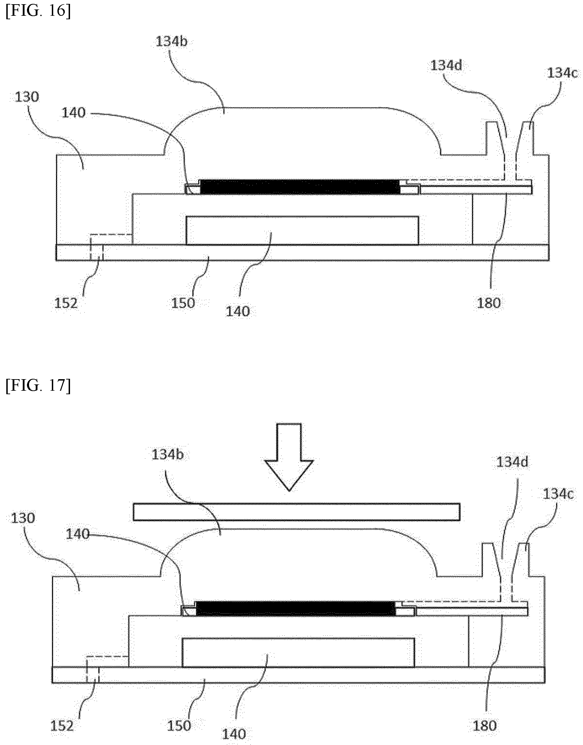

Referring to FIGS. 17, 18, 19, 20, and 21, when the window member disposed in the central region of the upper case 110 is pressed by the pressurization of the user or the pressurization of the machine, the well array 140 having the liquid sample filled in each of the plurality of microwells 142 is pressed against the CMOS photosensor array 150.

Accordingly, it is possible to prevent air pockets from being generated in the corners or edge regions of the well array 140. The air pockets expand or contract due to temperature changes during the PCR process, which causes errors in the test results. However, according to the present disclosure, when the PCR solution is moved by pumping the vacuum device and the solution fills the reaction space, the generation of air pockets in the corners or edge regions of the well array, which is the reaction space, is blocked, so that the generation of errors in the PCR test results due to air pockets may be prevented. In addition, since the well array is located above the CMOS photosensor array, real-time PCR reactions may be measured.

As described above, according to the present disclosure, since the well array of the PCR module is preloaded with reagents upon release, there is no need for a separate procedure to set up the reagents, drastically reducing the risk of contamination and a separate procedure for test preparation is not required

In addition, in the case of digital real-time PCR, even if the size of the plurality of microwells of the well array, which is the reaction space, is very small and the number of them is very large, it is possible to inject a sample into each of the reaction spaces.

In addition, since the sample is injected into the corner or edge region of the well array, air pockets are prevented from being generated in the corner or edge region of the well array, which is the reaction space, and the reliability of the test results is improved.

Hereinafter, a digital real-time PCR analysis method of the present disclosure, using the above-described cartridge for digital real-time PCR is described.

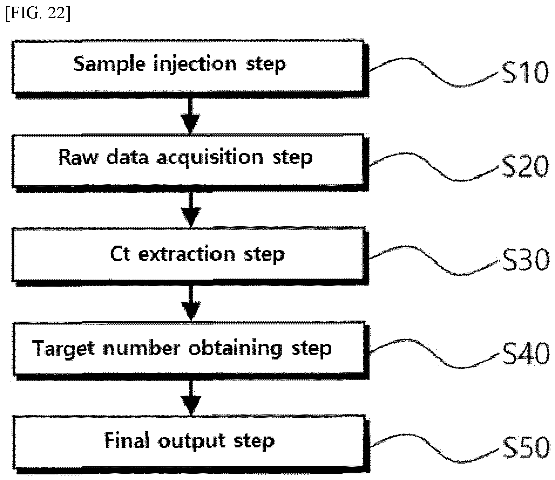

As shown in FIG. 22, the digital real-time PCR analysis method of the present disclosure may include:

a sample injection step (S10) of injecting the analysis target sample of the microfluidic chamber into each of the plurality of partitions;

a raw data acquisition step (S20) of acquiring raw data by capturing the reaction image of the analysis target sample filled in the plurality partitions in real-time through the CMOS photosensor array;

a cycle threshold (Ct) extraction step (S30) of extracting a plurality of Ct values for the plurality of partitions, respectively, from the raw data;

an individual partition target number obtaining step (S40) of obtaining a plurality of target numbers for the plurality of partitions, respectively, based on the plurality of Ct values; and

a final output step (S50) of obtaining a target concentration in the analysis target sample based on the plurality of target numbers.

In the sample injection step (S10), the microfluidic chamber may be formed of polydimethylsiloxane (PDMS) material. The plurality of partitions may correspond to the plurality of microwells 142 described above. In other words, one partition may be one microwell. The analysis target sample may be the liquid sample described above. As shown in FIGS. 7 to 21, in the sample injection step (S10), the analysis target sample may be injected into the plurality of partitions through the processes described above. At this time, it may be confirmed through the empty well algorithm that the injection of the analysis target sample into the plurality of partitions is complete. The image acquired after the arrival of the analysis target sample is confirmed may be utilized as raw data.

In other words, the raw data acquisition step (S20) may be performed after it is confirmed that the analysis target sample is filled in all of the plurality of partitions, and in the raw data acquisition step (S20), images for the plurality of partitions may be acquired as the raw data.

In the raw data acquisition step (S20), the raw data may be collected at least once per cycle. The cycle may be a temperature control time period provided to repeat denaturation, annealing, and elongation steps.

In the raw data acquisition step (S20), a region between partitions may be distinguished through a contour algorithm. In other words, a wall between microwells may be distinguished through the contour algorithm.

In the Ct extraction step (S30), a color variable of an image region for each of the plurality of partitions may be extracted to obtain a fluorescence intensity value. The fluorescence intensity value may be acquired as time series (cycle number) data. In other words, the fluorescence intensity value for each of the plurality of partitions may be acquired as real-time data (for each cycle). The color variable may include at least one or more of brightness, saturation, hue, contrast, and color code. For example, a partition image may be processed into a complementary color image, and a contrast value may be assigned to each pixel to extract it as a color variable. For another example, the saturation value of each pixel may be extracted as a color variable. For another example, the brightness value, saturation value, hue value, and contrast value may be extracted for each pixel, and the linear function value with independent weights for each variable may be extracted as a color variable.

In the Ct extraction step (S30), a fluorescence intensity function having the fluorescence intensity as a dependent variable and time (cycle number) as an independent variable may be acquired for each of the plurality of partitions. The fluorescence intensity function may be extracted from raw data. The Ct value may be a point (time or cycle number value) at which the second differentiation value of the fluorescence intensity function with respect to time (or cycle number) is at a maximum. The fluorescence intensity function may be a fitting function.

In the target number obtaining step (S40), the target number may be the copy number of the target gene in the analysis target sample. Each of the plurality of target numbers for the plurality of partitions may be obtained by the Equation 1.

[Equation 1]

$$N_i = 10^{\frac{Ct_i - Ct_{ref}}{a}},$$

where $N_i$ is the target number in the $i^{th}$ partition.

$Ct_i$ is the Ct value of the $i^{th}$ partition.

$Ct_{ref}$ is determined by at least one or more of the number of amplicons required to measure Ct and the fluorescence efficiency of the probe.

a is a constant determined by the PCR efficiency. The PCR efficiency may represent the rate at which the target gene is amplified by the PCR reaction. For example, a PCR efficiency of 100% means that the target is amplified 2-fold per PCR reaction cycle, and a PCR efficiency of 90% means that the target is amplified 1.8-fold per PCR reaction cycle. The relationship between a and the PCR efficiency Ep may be expressed as the following Equation 4.

[Equation 4]

$$Ep = -10^{\left(-\frac{1}{a}\right)} - 1$$

In general, the PCR efficiency may be set to 90% to 110%, and therefore a may be -3.103 to -3.587.

The above Equation 1 may be derived from the following Equation 5.

[Equation 5]

$$Ct_i = a \times log(N_i) + Ct_{ref}$$

The left side of the Equation 5 may be a value acquired in the Ct extraction step (S30).

The digital real-time PCR analysis method of the present disclosure acquires the value of $Ct_{ref}$ through a standard sample, so that analysis may be made even for ultra-high concentration samples that were previously impossible to analyze.

In the target number obtaining step (S40), the $Ct_{ref}$ may be obtained by the following Equation 2.

[Equation 2]

$$Ct_{ref} = a \times log\left(\frac{1}{V_{unit}}\right) + Ct_0 \quad ,$$

where Vunit is the volume of one partition. For example, it may mean the volume of one microwell, and may actually be the average volume of a plurality of microwells.

$Ct_0$ may be a Ct value acquired from a standard sample. Specifically, $Ct_0$ is a Ct value obtained by PCR amplification when the initial target number is 1 in all of the plurality of partitions. For example, if the number of partitions is 20,000, it may be a Ct value obtained by PCR amplification when the target number is 1 in a volume of 20,000 X Vunit. Ct0 is a value acquired by measuring a standard sample of a known concentration after stepwise dilution.

The concentration of the standard sample may be $10^7$ copies/rxn to $10^8$ copies/rxn. The standard sample may be an international standard material and a self-produced standard material. For example, the standard sample may be WHO biological reference materials (WHO international standards and reference materials).

In the final output step (S50), the concentration Con of the analysis target sample may be obtained by the following Equation 3.

[Equation 3]

$$Con = \sum_{i=1}^{n} N_i \quad ,$$

where n is the total number of the plurality of partitions.

In the final output step (S50), the negativity or positivity of the analysis target sample may be determined based on the Con value.

Although the embodiments according to the present disclosure have been described above, these are merely exemplary, and those skilled in the art will understand that various modifications and equivalent embodiments are possible therefrom. Therefore, the true technical protection scope of the present disclosure should be defined by the following claims.

Industrial Applicability

The digital real-time PCR analysis method of the present disclosure may provide a digital real-time PCR analysis method capable of performing digital real-time PCR analysis on high-concentration or low-concentration samples, i.e., on samples with a wide concentration range that exceeds existing measurement limits.

The digital real-time PCR analysis method of the present disclosure may implement digital PCR that provides real-time graphs for individual partitions, thereby eliminating false positive or false negative errors.

The digital real-time PCR analysis method of the present disclosure may obtain the same effect as repeatedly evaluating digital real-time PCR tens of thousands of times with one cartridge, and may obtain the initial concentration in the sample by calculating the number of targets present in each unit compartment based on the values measured in each unit compartment.

**Claims**

1. A digital real-time PCR analysis method using a cartridge for digital real-time PCR which comprises:

 a microfluidic chamber in which an analysis target sample of a liquid phase is stored;
 a well array attached to a bottom surface of the microfluidic chamber and supplied with the analysis target sample from the microfluidic chamber; and
 a CMOS photosensor array located on a bottom surface of the well array and configured to capture a reaction image of the analysis target sample filled in a plurality of partitions provided in the well array in real-time, the digital real-time PCR analysis method comprising:

 a sample injection step (S10) of injecting the analysis target sample of the microfluidic chamber into each of the plurality of partitions;
 a raw data acquisition step (S20) of acquiring raw data by capturing the reaction image of the analysis target sample filled in the plurality partitions in real-time through the CMOS photosensor array;
 a cycle threshold (Ct) extraction step (S30) of extracting a plurality of Ct values for the plurality of partitions, respectively, from the raw data;
 an individual partition target number obtaining step (S40) of obtaining a plurality of target numbers for the plurality of partitions, respectively, based on the plurality of Ct values; and
 a final output step (S50) of obtaining a target concentration in the analysis target sample based on the plurality of target numbers.

2. The digital real-time PCR analysis method of claim 1, wherein, in the sample injection step (S10), the microfluidic chamber is formed of polydimethylsiloxane (PDMS) material.

3. The digital real-time PCR analysis method of claim 1, wherein, in the sample injection step (S10), when the analysis target sample is injected into each of the plurality of partitions, the well array is completely in contact with the CMOS photosensor array.

4. The digital real-time PCR analysis method of claim 3, wherein the raw data acquisition step (S20) is performed after confirming that all of the plurality of partitions are filled with the analysis target sample, and in the raw data acquisition step (S20), images for the plurality of partitions are acquired as the raw data.

5. The digital real-time PCR analysis method of claim 4, wherein, in the raw data acquisition step (S20), the raw data is collected at least once per cycle.

6. The digital real-time PCR analysis method of claim 4, wherein, in the Ct extraction step (S30),

a fluorescence intensity function with fluorescence intensity as a dependent variable and time or cycle number as an independent variable is acquired for each of the plurality of partitions from the raw data, and

the Ct value is a time or cycle number value at which a second differentiation value of the fluorescence intensity function is at a maximum.

7. The digital real-time PCR analysis method of claim 6, wherein, in the target number obtaining step (S40),

each of the plurality of target numbers for the plurality of partitions is obtained by the following Equation 1:

[Equation 1]

$$N_i = 10^{\frac{Ct_i - Ct_{ref}}{a}} ,$$

where $N_i$ is the target number in an $i^{th}$ partition,
$Ct_i$ is the Ct value of the $i^{th}$ partition,
$Ct_{ref}$ is determined by at least one or more of an amplicon number required to measure Ct and a fluorescence efficiency of a probe, and
$a$ is a constant determined by a PCR efficiency.

8. The digital real-time PCR analysis method of claim 7, wherein, in the target number obtaining step (S40),

the $Ct_{ref}$ is obtained by the following Equation 2:

[Equation 2]

$$Ct_{ref} = a \times \log\left(\frac{1}{V_{unit}}\right) + Ct_0 ,$$

where $V_{unit}$ is a volume of one partition, and
$Ct_0$ is the Ct value acquired from a standard sample.

9. The digital real-time PCR analysis method of claim 8, wherein a concentration of the standard sample is $10^7$ copies/rxn to $10^8$ copies/rxn.

10. The digital real-time PCR analysis method of claim 7, wherein, in the final output step (S50),

a concentration Con of the analysis target sample is obtained by the following Equation 3:

[Equation 3]

$$Con = \sum_{i=1}^{n} N_i ,$$

where n is a total number of the plurality of partitions.

11. The digital real-time PCR analysis method of claim 10, wherein, in the final output step (S50), negativity or positivity of the analysis target sample is determined based on the Con value.

[FIG. 1]

[FIG. 2]

[FIG. 3]

[FIG. 4a]

[FIG. 4b]

[FIG. 5a]

[FIG. 5b]

[FIG. 5c]

[FIG. 6]

[FIG. 7]

[FIG. 8]

[FIG. 9]

[FIG. 10]

Air is pumped out

[FIG. 11]

Air is pumped out

[FIG. 12]

Air is pumped out

[FIG. 13]

Air is pumped out

[FIG. 14]

[FIG. 15]

[FIG. 16]

[FIG. 17]

[FIG. 18]

134b      134d

134c

130

140

152    150    140          180

[FIG. 19]

134b      134d

134c

130    140

152    150    140          180

[FIG. 20]

[FIG. 21]

[FIG. 22]

| Sample injection step | S10 |
| Raw data acquisition step | S20 |
| Ct extraction step | S30 |
| Target number obtaining step | S40 |
| Final output step | S50 |

## INTERNATIONAL SEARCH REPORT

International application No.

**PCT/KR2023/015053**

### A. CLASSIFICATION OF SUBJECT MATTER

**G01N 21/84**(2006.01)i; **G01N 21/25**(2006.01)i; **G06T 7/90**(2017.01)i; **G06V 10/10**(2022.01)i; **B01L 7/00**(2006.01)i; **C12Q 1/686**(2018.01)i; **G01N 21/17**(2006.01)i

According to International Patent Classification (IPC) or to both national classification and IPC

### B. FIELDS SEARCHED

Minimum documentation searched (classification system followed by classification symbols)

G01N 21/84(2006.01); B01L 7/00(2006.01); C12Q 1/6851(2018.01); C12Q 1/686(2018.01); G01N 35/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 미소유체 챔버(microfluid chamber), 웰 어레이(well array), CMOS 포토센서 어레이(CMOS photosensor array), 디지털 실시간 PCR(real-time digital PCR), Ct(cycle threshold)

### C. DOCUMENTS CONSIDERED TO BE RELEVANT

| Category* | Citation of document, with indication, where appropriate, of the relevant passages | Relevant to claim No. |
|---|---|---|
| Y | WILSON et al. EXTENDING DIGITAL PCR ANALYSIS BY MODELLING QUANTIFICATION CYCLE DATA. BMC BIOINFORMATICS. 17, 421 (2016), 12 October 2016. See abstract and page 1, left column, line 1 – page 4, left column, line 40. | 1-6 |
| A | | 7-11 |
| Y | KR 10-2185443 B1 (OPTOLANE TECHNOLOGIES INC.) 01 December 2020 (2020-12-01) See paragraph [0090], claims 1 and 3 and figures 2 and 6. | 1-6 |
| A | KR 10-2021-0094385 A (OPTOLANE TECHNOLOGIES INC.) 29 July 2021 (2021-07-29) See paragraphs [0037]-[0044] and figures 1 and 2. | 1-11 |
| A | KR 10-2019-0120947 A (LOGOS BIOSYSTEMS, INC.) 25 October 2019 (2019-10-25) See paragraphs [0026]-[0043] and figures 1-2. | 1-11 |
| A | KR 10-2019-0096614 A (DANKOOK UNIVERSITY CHEONAN CAMPUS INDUSTRY ACADEMIC COOPERATION FOUNDATION) 20 August 2019 (2019-08-20) See paragraphs [0029]-[0061] and figures 1-2. | 1-11 |

☐ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **26 January 2024** | **26 January 2024** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2023/015053**

| Patent document cited in search report | | | Publication date (day/month/year) | Patent family member(s) | | | Publication date (day/month/year) |
|---|---|---|---|---|---|---|---|
| KR | 10-2185443 | B1 | 01 December 2020 | CN | 110396472 | A | 01 November 2019 |
| | | | | CN | 110396472 | B | 25 July 2023 |
| | | | | EP | 3560593 | A1 | 30 October 2019 |
| | | | | JP | 2019-187414 | A | 31 October 2019 |
| | | | | JP | 6721194 | B2 | 08 July 2020 |
| | | | | KR | 10-2019-0124134 | A | 04 November 2019 |
| | | | | US | 11426731 | B2 | 30 August 2022 |
| | | | | US | 2019-0329254 | A1 | 31 October 2019 |
| KR | 10-2021-0094385 | A | 29 July 2021 | KR | 10-2021-0094436 | A | 29 July 2021 |
| | | | | KR | 10-2329337 | B1 | 19 November 2021 |
| | | | | KR | 10-2568295 | B1 | 18 August 2023 |
| KR | 10-2019-0120947 | A | 25 October 2019 | KR | 10-2133633 | B1 | 13 July 2020 |
| | | | | US | 2021-0269867 | A1 | 02 September 2021 |
| | | | | WO | 2019-203552 | A1 | 24 October 2019 |
| KR | 10-2019-0096614 | A | 20 August 2019 | KR | 10-2069337 | B1 | 22 January 2020 |

Form PCT/ISA/210 (patent family annex) (July 2022)

31

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220125179
- KR 1020230130511